# EUROPÄISCHE PATENTSCHRIFT

(11) **EP 1 648 566 B1**
(45) Veröffentlichungstag und Bekanntmachung des Hinweises auf die Patenterteilung: **14.05.2008**
(21) Anmeldenummer: 04712497.9
(22) Anmeldetag: 19.02.2004
(51) Int. Cl.: A61K 36/00, A61K 31/35, A61P 17/06

(54) **VERFAHREN ZUR HERSTELLUNG VON FLAVONOID-HALTIGEN ZUSAMMENSETZUNGEN UND IHRE VERWENDUNG**
METHOD FOR THE PRODUCTION OF FLAVONOID-CONTAINING COMPOSITIONS AND USE THEREOF
PROCEDE POUR PRODUIRE DES COMPOSITIONS CONTENANT DES FLAVONOIDES ET LEUR UTILISATION

(30) Priorität: 26.02.2003 DE 10308162
(43) Veröffentlichungstag der Anmeldung: 26.04.2006
(73) Patentinhaber: Universitätsklinikum Freiburg, 79106 Freiburg (DE); NIG Nahrungs-Ingenieurtechnik GmbH, 39124 Magdeburg (DE)
(72) Erfinder: SCHEMPP, Christoph, Mathis, 79104 Freiburg (DE); WÄHLING, Axel, 39120 Magdeburg (DE); LANGE, Elfriede, 39118 Magdeburg (DE)
(74) Vertreter: Kalhammer, Georg
(86) Internationale Anmeldenummer: PCT/EP2004/001598
(87) Internationale Veröffentlichungsnummer: WO 2004/075816

(56) Entgegenhaltungen:
- WO-A-97/14319
- US-B1- 6 409 996
- US-B2- 6 488 923

## Beschreibung

Die vorliegende Erfindung betrifft ein Verfahren zur Herstellung von Flavonoid-haltigen Zusammensetzungen aus Pflanzen der Familie der Resedaceae. Die Zusammensetzungen und bei der Herstellung anfallende Zwischenprodukte können vorteilhaft verwendet werden zum Färben von Naturtextilien und Leder sowie zur Herstellung von Farbpigmenten. Die Zusammensetzungen und bei der Herstellung anfallenden Zwischenprodukte können auch in der Kosmetik und als Arzneimittel eingesetzt werden.

Die Familie der Resedaceae umfaßt unter anderem die Pflanzenarten *Reseda alba L., Reseda glauca L., Reseda lutea L., Reseda odorata L., Reseda phyteuma L. und Reseda luteola L.* Seit Jahrhunderten wird *Reseda luteola,* auch Färberwau genannt, zur Färbung von Naturtextilien in Gelb- und Grüntönen auch in Kombination mit anderen Färberpflanzen verwendet. Bis ins 20. Jahrhundert benutzte man die Pflanze zum Färben von Seide. H. Schweppe (Handbuch der Naturfarbstoffe, ecomed Verlag, 1993) beschreibt die Anwendung, Analytik und historische Entwicklung der Verwendung dieser Pflanze in der Textilfärbung. Später wurde der Farbstoff vollständig von synthetischen Farbstoffen verdrängt.

*Reseda luteola* läßt sich leicht kultivieren, die Pflanze bevorzugt kalkhaltige Böden. Neuere Forschungsarbeiten von Vetter und Biertümpfel (Tagungsbände 1997 und 1999 Forum Färbepflanzen; FNR Gülzow) zum Anbau von *Reseda luteola* in Deutschland stellen das landwirtschaftliche Potential als nachwachsenden Rohstoff dar.

*Reseda luteola* enthält neben den Hauptkomponenten Zucker, Eiweiße und Ballaststoffe sekundäre Pflanzeninhaltsstoffe, wobei die Flavonoide von Interesse sind. Nachgewiesen wurden unter anderem die Flavonoide Luteolin, Apigenin, Luteolin-7-glucosid und Luteolin-3,7-glucosid (H. Schweppe, Handbuch der Naturfarbstoffe, ecomed Verlag, 1993; L. Adam, Tagungsband Forum Färberpflanzen 1999, K. Loest, Abschlußbericht zum Forschungsprojekt FNR 97NR147-F, 2001).

Das in *Reseda luteola* enthaltene Luteolin stellt das färbende Prinzip dar. Es gehört zur Gruppe der Flavonoide, das sind Pflanzeninhaltsstoffe mit Verbreitung im ganzen Pflanzenreich. Flavonoide weisen eine Vielzahl biologischer Aktivitäten auf. Beispielsweise wurde für Luteolin eine cardioprotektive Wirkung berichtet, indem es die Oxidation von Lipiden verhindert und die Cholesterinsynthese hemmt (Cook et al. (1996) Nutritional Biochemistry 7, 66; Pietta (2000) J. Nat. Prod. 63, 1035). Weiterhin ist eine analgetische und antiinflammatorische Wirkung von Luteolin bekannt (Toreda et al. (1994) Z. Naturforschung, C49, 35). Außerdem wurden Mastzell-stabilisierende Wirkungen von Flavonoiden wie Luteolin und Quercetin beschrieben (Kimata et al. (2000) Chemical and Experimental Allergy, 30, 501). In vitro wurden zahlreiche zytostatische Effekte von Luteolin und anderen Flavonoiden beschrieben.

Weiterhin sind eine antibakterielle Wirkung von Luteolin (Pettit et al. (1996) J. Ethnopharmacol. 53, 57) und eine antioxidative Wirkung von Flavonoiden (Pietta (2000) J. Nat. Prod. 63, 1035) bekannt. WO 00/26206 A1 beschreibt ein Verfahren zur Herstellung von Luteolin und Derivaten davon. Im Bereich der dermatologischen Kosmetik werden verschiedene Antioxidantien wie Vitamin C und Vitamin E verwendet. Außerdem finden Retinoide Verwendung zur Verzögerung der lichtbedingten Hautalterung. Pflanzenextrakte aus *Potentilla erecta* verbessern womöglich die Verankerung der Epidermis in der Basalmembran und können zu einer Straffung der Haut führen (WO 98/19664).

Die Färbung mit Pflanzenfarben beschränkt sich derzeit auf den handwerklichen und kunsthandwerklichen Bereich. Dabei werden wäßrige Abkochungen der Pflanzenteile zum Färben verwendet. Diese Verfahrensweise hat zwei entscheidende Nachteile. Die Farbstoffe der Pflanzen werden nur ungenügend gelöst, da insbesondere die Aglykone Luteolin und Apigenin schwer wasserlöslich sind. Ein weiterer Nachteil ist die Verwendung der Pflanzenteile, die in industriell arbeitenden Veredelungsbetrieben auf Hochleistungsmaschinen nicht möglich ist. Die synthetische Herstellung von Luteolin ist aufwendig und teuer. FR 2 632 523 A1 offenbart die Extraktion verschiedener Pflanzen mit ethanolischen Lösungen, Wasser oder Hexan.

Eine Aufgabe der vorliegenden Erfindung ist es, eine Flavonoid-haltige Zusammensetzung mit standardisiertem Flavonoidgehalt, insbesondere Luteolingehalt, bereitzustellen.

Überraschenderweise wurde gefunden, daß Zusammensetzungen mit einem hohen Luteolingehalt erhalten werden, wenn man vor einer Extraktion von Resedapflanzen mit einem Lösungsmittel das Pflanzenmaterial mit einer wäßrigen Lösung vorextrahiert. Die Erfindung betrifft daher ein Verfahren zur Herstellung einer flavonoidhaltigen Zusammensetzung, das umfasst, dass man a) Material wenigstens einer Pflanze der Resedaceae einer Vorextraktion mit einer wässrigen Lösung unterzieht, b) den in Schritt a) erhaltenen Rückstand an Pflanzenmaterial mit einem ersten Lösungsmittel extrahiert, das eine Lösung mit einem Anteil von 15 bis 100 % an polarem organischen Lösungsmittel in Wasser ist, und c) wenigstens einen Teil des ersten Lösungsmittels aus dem in Schritt b) erhaltenen Extrakt entfernt, um eine Zusammensetzung mit einem Luteolingehalt von wenigstens 10 % zu erhalten.

Das eingesetzte Pflanzenmaterial kann von jeder beliebigen Pflanze der Familie der Resedaceae stammen. Beispiele sind die Pflanzenarten *Reseda alba L., Reseda glauca L., Reseda lutea L., Reseda odorata L., Reseda phyteuma L. und Reseda luteola L.* Bevorzugt ist *Reseda luteola L.* Es können auch Gemische aus Materialien mehrerer Pflanzenarten eingesetzt werden. Bei dem Material kann es sich um beliebige Teile der Pflanzen handeln, bevorzugt handelt es sich aber um die oberirdischen Pflanzenteile, z.B. Stengel, Blätter, Samen und/oder Blüten. Das Material kann dabei in verschiedenster Form eingesetzt werden, beispielsweise als frisches, noch unbearbeitetes Material. Vorteilhaft ist es aber, wenn das Material in getrockneter Form eingesetzt wird. Das Pflanzenmaterial kann dabei bereits zerkleinert vorliegen.

Nach einer Ausführungsform der Erfindung wird das Pflanzenmaterial vor der Extraktion mit dem ersten Lösungsmittel einer Vorextraktion mit einer wäßrigen Lösung unterzogen und der dann erhaltene Rückstand an Pflanzenmaterial weiterverarbeitet. Die wäßrige Lösung besteht zu wenigstens 80%, bevorzugt zu wenigstens 85%, bevorzugter zu wenigstens 90%, noch bevorzugter zu wenigstens 95%, noch bevorzugter zu wenigstens 98%, aus Wasser. Am bevorzugtesten wird die Vorextraktion mit Wasser durchgeführt. Dabei kann es sich um Leitungswasser oder destilliertes Wasser oder Wasser aus anderer Quelle handeln. Die Temperatur der wäßrigen Lösung ist vorzugsweise 15 bis 90°C, bevorzugter 15 bis 80°C, am bevorzugtesten ungefähr 15 bis 40°C. Der pH-Wert der wäßrigen Lösung ist nicht besonders eingeschränkt, vorzugsweise wird er durch Zugabe von entsprechenden Reagenzien auf pH 4,5 bis 9, bevorzugter auf pH 5,5 bis 8, eingestellt. Die wäßrige Lösung kann Salze oder Puffersubstanzen enthalten. Weiterhin können in geringen Mengen andere Lösungsmittel, wie z.B. polare organische Lösungsmittel, enthalten sein. Die Vorextraktion wird in der Regel erschöpfend durchgeführt. Der wäßrige Vorextrakt kann zu färberischen Zwecken oder zu pharmazeutischen Zwecken eingesetzt werden. Der wässrige Vorextrakt
kann dem Fachmann bekannten Trocknungsverfahren unterworfen werden. Das vorextrahierte Pflanzenmaterial wird dann mit einem ersten Lösungsmittel extrahiert.

Das erste Lösungsmittel ist eine Lösung mit einem Anteil von 15 bis 100% an polarem organischem Lösungsmittel in Wasser. Soweit nicht anders angegeben, sind %-Angaben in dieser Anmeldung Gewichts-%. Das erste Lösungsmittel kann somit ein im wesentlichen reines polares organisches Lösungsmittel ohne Wasser sein (100%). Es kann aber auch ein Gemisch aus einer wäßrigen Lösung und wenigstens einem polaren organischen Lösungsmittel sein.

Polare organische Lösungsmittel im Sinne der vorliegenden Anmeldung umfassen Alkohole, Ketone, Carbonsäuren, Ester, Amide, Aldehyde, Nitrile, Nitroverbindungen, Sulfoxide und organische Verbindungen mit einer Dielektrizitätskonstante (20°C) von mindestens 2,5, vorzugsweise mindestens 5, am bevorzugtesten mindestens 10. Vorzugsweise ist das polare organische Lösungsmittel in jedem Verhältnis mit Wasser mischbar.

Polare organische Lösungsmittel im Sinne der vorliegenden Erfindung sind nicht besonders eingeschränkt, vorzugsweise handelt es sich um Alkohole oder Ketone. Besonders bevorzugt sind Alkohole mit 1 bis 4 Kohlenstoffatomen oder Ketone mit 1 bis 4 Kohlenstoffatomen. Beispiele für organische Lösungsmittel sind Methanol, Ethanol, 1-Propanol, Isopropanol, 1-Butanol, 2-Butanol, Ethylenglycol, Aceton, Butanon oder beliebige Mischungen der genannten Lösungsmittel. Am bevorzugtesten sind die polaren organischen Lösungsmittel Methanol, Ethanol Isopropanol oder Aceton.

Das erste Lösungsmittel, das in Schritt b) eingesetzt wird, ist ein Gemisch aus einer wäßrigen Lösung und wenigstens einem polaren organischen Lösungsmittel. Dabei hat in der Regel das polare organische Lösungsmittel in dem Gemisch einen Anteil von 15 bis 95%. Bevorzugt ist ein Anteil des polaren organischen Lösungsmittels von 20 bis 90%, bevorzugter von 30 bis 80%, am bevorzugtesten von 40 bis 70%. Bevorzugte polare organische Lösungsmittel in dem Gemisch entsprechen den oben angegebenen bevorzugten polaren organischen Lösungsmitteln. Besonders bevorzugt sind ein Wasser/Alkohol- oder ein Wasser/Keton-Gemisch.

Die Extraktion kann mittels bekannter Verfahren erfolgen, beispielsweise durch Mazeration, Perkolation oder Digestion. Die Extraktion kann kontinuierlich, diskontinuierlich, einstufig oder mehrstufig erfolgen (Pharmazeutisches Wörterbuch, 1993, 7. Auflage, de Gryter Verlag, Berlin). Am Ende wird üblicherweise ungelöstes Pflanzenmaterial abgetrennt.

Die Temperatur bei der Extraktion beträgt üblicherweise 20 bis 95°C, vorzugsweise 20 bis 80°C, am bevorzugtesten 30 bis 70°C. Die Temperatur bei der Extraktion wird am leichtesten dadurch eingestellt, daß das Extraktionsmittel vor der Extraktion auf die gewünschte Temperatur gebracht wird. Dem Fachmann ist klar, daß die Obergrenze der Temperatur von dem Siedepunkt des ersten Lösungsmittels oder des polaren organischen Lösungsmittels in dem Gemisch bestimmt wird.

Der so erhaltene Pflanzenextrakt kann konzentriert werden, indem wenigstens ein Teil des ersten Lösungsmittels entfernt wird. Dies kann auf verschiedenste Weisen erfolgen. Der Begriff "Extrakt" bezeichnet eine Zusammensetzung, die durch ein Extraktionsverfahren erhältlich ist.

Beispielsweise kann der erhaltene Extrakt vorteilhaft unter Vakuum eingeengt werden, so daß wenigstens ein Teil des polaren organischen Lösungsmittels aus dem Extrakt entfernt wird. Dabei kann sich in der Vorlage nach Abkühlung ein Niederschlag bilden. Dieser kann durch Filtration, Separation oder andere geeignete Verfahrenstechniken gewonnen werden. Die nach der Destillation verbleibende wäßrige Phase kann auch ohne Abtrennung des Niederschlags komplett durch Konzentrierung und/oder Trocknung aufgearbeitet werden. Die Trocknung des separierten Niederschlags oder der kompletten wäßrigen Phase kann mittels bekannter Trocknungsverfahren wie z.B. Sprühtrocknung, Wirbelschichttrocknung, Gefriertrocknung oder Vakuumtrocknung erfolgen, um das Produkt als Pulver zu erhalten. Die Trocknung kann sowohl ohne als auch unter Zugabe von Trocknungshilfsmitteln wie z.B. Glukosesirup oder Maltodextrin erfolgen. Die Art der Trocknungshilfsmittel ist aber nicht besonders eingeschränkt.

Es ist ebenfalls möglich, eine Präzipitation herbeizuführen, z. B. durch Abkühlen, Zugabe von Wasser oder Ansäuern der Lösung. Der sich bildende Niederschlag stellt dann das in Schritt c) erhaltene Produkt dar und kann nach üblichen Verfahren gewonnen werden. Spätestens bei der Gewinnung des Niederschlags wird wenigstens ein Teil des ersten Lösungsmittels entfernt.

In der Regel findet in Schritt c) eine Volumenreduktion des Extrakts aus Schritt b) statt. Vorzugsweise wird das Volumen des Extrakts auf weniger als 50% des ursprünglichen Volumens reduziert, bevorzugter auf weniger als 10%, am bevorzugtesten auf weniger als 5%. Das in Schritt c) erhaltene Produkt kann ein Feststoff, z. B. ein Pulver, ein Konzentrat oder eine Suspension sein.

Nach Schritt b) und c) erhält man bereits eine Flavonoid-angereicherte Zusammensetzung.

Überraschenderweise wurde gefunden, daß Zusammensetzungen mit einem noch höheren Luteolingehalt erhalten werden, wenn nach der Extraktion mit dem ersten Lösungsmittel der erhaltene Extrakt konzentriert, in einem Lösungsmittel aufgenommen und anschließend ausgefällt wird.

Gemäß dieser Ausführungsform wird das in Schritt c) erhaltene Produkt in einem zweiten Lösungsmittel aufgenommen und anschließend eine Präzipitation von Flavonoidangereichertem Produkt herbeigeführt, so daß ein Feststoff erhalten wird. Die Präzipitation kann durch Ansäuern, Abkühlen und/oder Zugabe von Wasser herbeigeführt werden. Diese Maßnahmen können einzeln oder kombiniert angewendet werden.

Das zweite Lösungsmittel ist eine Lösung mit einem Anteil von 0 bis 100% an polarem organischem Lösungsmittel in Wasser. Das zweite Lösungsmittel kann eine wäßrige Lösung ohne polares organisches Lösungsmittel sein (0%). Es kann auch ein im wesentlichen reines polares organisches Lösungsmittel sein (100%). Schließlich kann wiederum auch ein Gemisch aus wäßriger Lösung und mindestens einem polaren organischen Lösungsmittel eingesetzt werden. Die einsetzbaren und bevorzugten polaren organischen Lösungsmittel, wie sie oben für das erste Lösungsmittel beschrieben wurden, gelten auch für das zweite Lösungsmittel.

Das in Schritt c) erhaltene Produkt wird soweit möglich in dem zweiten Lösungsmittel gelöst. Dem Fachmann ist bewußt, daß die Löslichkeit durch den pH-Wert und/oder die Temperatur beeinflußt wird. So kann der pH-Wert des zweiten Lösungsmittels verändert werden. Er wird vorzugsweise auf einen pH-Wert von 5 bis 12 eingestellt, vorzugsweise von 6 bis 10, bevorzugter von 7 bis 8. Die Einstellung des pH-Werts kann durch geeignete Puffer oder Alkalien erfolgen, wobei Alkalien bevorzugt sind, z.B. Natronlauge, Ammoniak oder Natriumhydrogencarbonat. Die Temperatur des zweiten Lösungsmittels kann höher als Raumtemperatur sein. So kann das zweite Lösungsmittel auf eine Temperatur von 30 bis 100°C eingestellt werden, bevorzugt auf 40 bis 80°C, bevorzugter auf 50 bis 80°C.

Nach dem Lösen des Produkts in dem zweiten Lösungsmittel - soweit es möglich ist - können gegebenenfalls unlösliche Bestandteile abgetrennt werden. Dazu können allgemein bekannte Verfahren wie Filtration, Zentrifugation, etc. eingesetzt werden. Nach dem Lösen des Produkts in dem zweiten Lösungsmittel - soweit es möglich ist - kann durch verschiedene Maßnahmen erreicht werden, daß sich ein Flavonoid-angereicherter Niederschlag bildet. Diese Maßnahmen umfassen vor allem Ansäuern der Lösung, Abkühlen der Lösung und/oder Zugabe von Wasser. Diese Maßnahmen können einzeln oder kombiniert angewendet werden.

Wird die Lösung nach dem Lösen angesäuert, so geschieht dies vorzugsweise durch Zugabe von HCl. Der dadurch eingestellte pH-Wert ist vorzugsweise 1 bis 6, bevorzugter 2,5 bis 6, am bevorzugtesten 3 bis 5.

Wird die Lösung abgekühlt, so ist die eingestellte Temperatur unter 20°C, vorzugsweise unter 15°C, bevorzugter unter 10°C. Die Lösung wird üblicherweise nicht unter 0°C abgekühlt, vorzugsweise nicht unter 4°C. Die angegebenen Ober- und Untergrenzen der Temperatur können beliebig miteinander kombiniert werden.

Wird Wasser zu der Lösung gegeben, so handelt es sich in der Regel um destilliertes Wasser. Es kann aber auch nicht destilliertes Wasser sein. Ebenso kann das Wasser eine wäßrige Lösung mit einer oder mehreren Substanzen sein, z. B. können Salze, Puffer oder andere Substanzen enthalten sein. Das Volumenverhältnis Lösung:Wasser ist in der Regel 1:100 bis 100:1, bevorzugt 1:10 bis 10:1, am bevorzugtesten 1:3 bis 3:1.

Handelt es sich bei der Flüssigkeit, in der das in Schritt c) erhaltene Produkt aufgenommen wird, um eine wäßrige Lösung ohne polares organisches Lösungsmittel, so hat diese üblicherweise einen pH-Wert von 6 bis 10. Der pH-Wert kann durch Zugabe von Alkalien eingestellt werden. Vorzugsweise hat die wäßrige Lösung einen pH-Wert von 7 bis 8. Die Temperatur der wäßrigen Lösung ist üblicherweise 20 bis 80°C, vorzugsweise 40 bis 75°C, bevorzugter mehr als 50°C bis 70°C. Die wäßrige Lösung wird mit dem in Schritt c) erhaltenen Produkt, bei dem es sich um ein Pulver oder ein Konzentrat handeln kann, in Kontakt gebracht. Dabei löst sich wenigstens ein Teil des Produkts. Die unter diesen Bedingungen gegebenenfalls unlöslichen Bestandteile können über Filtration, Zentrifugation, Separation, Dekantieren oder andere übliche Trennmethoden abgetrennt werden. Die sodann erhaltene Lösung wird auf einen sauren pH-Wert eingestellt. Der saure pH-Wert ist vorzugsweise 2,5 bis 6,0, bevorzugter 3,0 bis 5,0. Die Einstellung des sauren pH-Werts erfolgt üblicherweise durch Zugabe von HCl. Dadurch bildet sich ein Präzipitat. Vorzugsweise bildet sich das Präzipitat nach Abkühlen der Lösung. In diesem Fall kann die Lösung auf weniger als 20°C abgekühlt werden, bevorzugt auf 4 bis 15°C, bevorzugter auf 4 bis 10°C. Der sich bildende Niederschlag kann wie beschrieben gewonnen werden. Dieser Niederschlag enthält in der Regel 25 bis 30% Luteolin. Sofern nicht anders angegeben, beziehen sich Angaben über den Luteolingehalt jeweils auf die Trockensubstanz der Extrakte (Lösungen, Konzentrate oder Pulver).

Gemäß einer weiteren Ausführungsform wird das in Schritt c) erhaltene Produkt mit einer wäßrigen Lösung ohne polares organisches Lösungsmittel bei Temperaturen von 30 bis 100°C, vorzugsweise 50°C bis 90°C, am bevorzugtesten 60°C bis 80°C aufgenommen. Das Konzentrat wird in dieser erhitzten Lösung soweit wie möglich gelöst. Nach dem Abkühlen der Lösung bildet sich ein Niederschlag, der entsprechend gewonnen und getrocknet werden kann. Vorzugsweise wird auf unter 20°C abgekühlt, bevorzugter auf unter 15°C, am bevorzugtesten auf unter 10°C. Gemäß dieser Ausführungsform muß zur Bildung des Niederschlags nicht angesäuert werden.

In einer anderen Ausführungsform enthält das zweite Lösungsmittel ein polares organisches Lösungsmittel. Es handelt sich üblicherweise um eine Lösung mit einem Anteil von 5-100% an polarem organischem Lösungsmittel in Wasser. Es wurde gefunden, daß durch ein Gemisch, das 50 bis 100% polares organisches Lösungsmittel in einer wäßrigen Lösung enthält, eine besondere Konzentrationserhöhung an Luteolin erzielt werden kann. Vorzugsweise hat das Gemisch einen Anteil an polarem organischem Lösungsmittel von 60 bis 100%, am bevorzugtesten von 70 bis 100%. Das polare organische Lösungsmittel ist vorzugsweise ein Alkohol. Das Gemisch hat in der Regel eine Temperatur von 20 bis 80°C, vorzugsweise hat es eine Temperatur, die der Siedetemperatur des polaren organischen Lösungsmittels entspricht oder bis zu 10°C darunter liegt. Nachdem das in Schritt c) erhaltene Produkt mit dem Gemisch (zweiten Lösungsmittel) in Kontakt gebracht worden ist und soweit möglich gelöst wurde, wird Wasser zu der gegebenenfalls klarfiltrierten Lösung gegeben. Dabei bildet sich ein Präzipitat, das insbesondere die Aglykone enthält. Diese Niederschläge können dann wie oben beschrieben aufgearbeitet werden. Gemäß dieser Ausführungsform können Zusammensetzungen mit einem Luteolingehalt von etwa 30 bis 40% erhalten werden. Wird die Zusammensetzung vor der Zugabe von Wasser abgekühlt, so kann sich ein leichter Niederschlag bilden, der gegebenenfalls abgetrennt werden kann. Weiterhin kann vor der Zugabe von Wasser das polare organische Lösungsmittel teilweise oder vollständig entfernt werden, z.B. durch Destillation. Der Rückstand wird dann mit Wasser verdünnt, wobei sich das Präzipitat bildet.

In einer besonderen Ausführungsform kann zur Ausfällung eine Abkühlung der Lösung und die Zugabe von Wasser kombiniert werden. Dadurch können ebenfalls Zusammensetzungen mit etwa 30 bis 40% Luteolin erhalten werden.

Eine weitere Erhöhung insbesondere der Aglykonanteile der Zusammensetzung kann dadurch erreicht werden, daß das in Schritt e) erhaltene Produkt nochmals in einem Gemisch aus einer wäßrigen Lösung und wenigstens einem polaren organischen Lösungsmittel aufgenommen wird. Dieses Lösen erfolgt vorteilhaft bei Temperaturen von 30 bis 80°C. Durch Zugabe von Wasser zum entsprechenden Lösungsmittelextrakt gelang es, einen Niederschlag mit weiter angereichertem Flavonoidgehalt zu gewinnen. Dieser Niederschlag kann wie beschrieben gewonnen und getrocknet werden. Dieses Produkt enthält etwa 35 bis 45% Luteolin. Dieser Schritt kann mit dem auf diese Weise erhaltenen Produkt nochmals wiederholt werden.

Es wurde auch gefunden, daß bei Durchführung der Schritte d) und e) auf eine Vorextraktion des Pflanzenmaterials mit einer wäßrigen Lösung verzichtet werden kann.

Die Erfindung betrifft daher auch ein Verfahren zur Herstellung einer Flavonoid-haltigen Zusammensetzung mit einem Luteolingehalt von wenigstens 10 %, das umfasst, dass man a) Material wenigstens einer Pflanze der Familie der Resedaceae mit einem ersten Lösungsmittel extrahiert, das eine Lösung mit einem Anteil von 15 bis 100 % an polarem organischen Lösungsmittel in Wasser ist, b) wenigstens einen Teil des ersten Lösungsmittels aus dem in Schritt a) erhaltenen Extrakt entfernt; c) das in Schritt b) erhaltene Produkt in einem zweiten Lösungsmittel aufnimmt und gegebenenfalls unlösliche Bestandteile abtrennt; und d) die in Schritt c) erhaltene Lösung ansäuert, abkühlt und/oder mit Wasser versetzt, so daß ein Feststoff erhalten wird. Für die einzelnen Schritte gelten die oben beschriebenen Varianten und bevorzugten Ausführungsformen entsprechend.

Die Erfindung betrifft weiterhin Flavonoid-haltige Zusammensetzungen, die durch ein Verfahren gemäß der vorliegenden Erfindung erhältlich sind. Die so erhaltenen Pflanzenextrakte und Flavonoid-haltigen Zusammensetzungen können vorteilhaft zum Färben von Textilien und Leder sowie zur Herstellung von Farbpigmenten verwendet werden. Sie lassen sich auch in der Kosmetik und in Arzneimitteln einsetzen.

Durch das erfindungsgemäße Verfahren ist es möglich, Flavonoid-haltige Zusammensetzungen (Pflanzenextrakte) mit standardisiertem Flavonoidgehalt, insbesondere Luteolingehalt, bereitzustellen. Die so erhaltenen Zusammensetzungen (Pflanzenextrakte) weisen einen hohen Luteolingehalt von wenigstens 10%, vorzugsweise wenigstens 20%, bevorzugter wenigstens 30%, noch bevorzugter wenigstens 35%, am bevorzugtesten wenigstens 40%, Luteolin auf. Derartige Zusammensetzungen oder Pflanzenextrakte sind ebenfalls Gegenstand der Erfindung. Die Extrakte enthalten vorzugsweise weitere Flavonoide, wie z.B. Apigenin, Luteolin-7-glucosid oder. Luteolin-3-7-glucosid.

Die erfindungsgemäßen Flavonoid-haltigen Zusammensetzungen haben vorzugsweise einen Flavonoidgehalt von 20 bis 90 %, bezogen auf die Gesamtzusammensetzung. Bevorzugter ist der Flavonoidgehalt höher als 20%, noch bevorzugter höher als 50%, am bevorzugtesten höher als 60%.

Weiterhin wurde überraschenderweise gefunden, daß durch eine Behandlung mit Extrakten aus der Pflanze *Reseda luteola* (Färberwau) unerwünschte Hautzustände signifikant verbessert werden können und eine Straffung und Glättung der Haut erreicht werden kann. Insbesondere wird durch eine lokale Verabreichung von Resedaextrakt eine signifikante Verbesserung der Symptome der Neurodermitis erreicht.

Ein weiterer Gegenstand der Erfindung ist daher die Verwendung eines Extrakts wenigstens einer Pflanze der Familie der Resedaceae zur Behandlung oder Vorbeugung von unerwünschten Zuständen der Haut, wie in den Ansprüchen definiert.

Ein Aspekt der Erfindung ist die Verwendung von Resedaceae-Extrakten zur Behandlung von Hauterkrankungen. Dazu zählen unter anderem Neurodermitis, Erytheme, Verbrennungen, Lichen ruber, Prurigo, Psoriasis, Pemphigus, Pemphigoid, Dermatitis herpetiformis, Sklerodermie, Lichen sclerosus, Dermatitis solaris, Morbus Favre Racouchaud, aktinische Keratosen, Elastosis cutis, Akne vulgaris, Follikulitis simplex und Rosacea. Die Erfindung betrifft auch die Verwendung von Resedaceae-Extrakten zur Herstellung eines Medikaments zur Behandlung von Hauterkrankungen.

Ein weiterer Aspekt der Erfindung ist die Verwendung von Resedaceae-Extrakten als Kosmetikum. Bevorzugte Verwendungen dieser Art sind zur Glättung der Haut, zur Straffung der Haut, zur Vorbeugung von Falten, zur Verringerung von Falten, zur Verringerung der Faltentiefe, zur Vorbeugung von Altershaut, zur Behandlung von Altershaut, zur Verbesserung des ästhetischen Eindrucks der Haut, zur Vorbeugung von durch Sonnenlicht induzierter Hautalterung, zur Vorbeugung von umweltbedingten Hautveränderungen, zur Behandlung von umweltbedingten Hautveränderungen etc. Die Erfindung betrifft auch die Verwendung von Resedaceae-Extrakten zur Herstellung eines Kosmetikums.

Dementsprechend bezieht sich die Erfindung auf die Verwendung eines Extraktes aus der Pflanze *Reseda luteola* zur Verbesserung der Hautstraffheit oder zur Verzögerung oder Bekämpfung der Hautalterung. Dazu kann dieses in eine kosmetische Zubereitung mit einem kosmetisch verträglichen Vehikel eingearbeitet werden. Die Zubereitung ist insbesondere nützlich, um eine Glättung der Haut zu erreichen, Zeichen von Hautalterungen zu verzögern oder vorzubeugen, die Ausbildung von Falten oder deren Tiefe zu verringern und Hautreizungen zu lindern. Die Zubereitungen können als Antifaltenprodukte, als Produkte gegen Hautalterung und als Hautpflegeprodukte verwendet werden.

Die kosmetischen Zubereitungen der vorliegenden Erfindung eignen sich zur Verzögerung der Hautalterung und zur Bekämpfung der Hautalterung, insbesondere der durch Sonneneinstrahlung bedingten Hautalterung. Die genannten kosmetischen Zubereitungen eignen sich darüber hinaus zur Behandlung oder Vorbeugung von Hautreizungen, wie Sonnenbrand und Erythemen der Haut.

Eine besonders geeignete Verwendung der genannten kosmetischen oder pharmazeutischen Präparationen liegt in der Verwendung zur Behandlung von Hauterkrankungen. Besonders geeignet sind die erfindungsgemäßen Zubereitungen zur Behandlung von Neurodermitis.

Die vorliegende Erfindung beschreibt auch Verfahren zur Herstellung von Reseda-Extrakten mit einem besonders hohen Gehalt an Flavonoiden. In diesen Präparationen liegt der Flavonoid-Gehalt zwischen 10 - 90 %, vorzugsweise zwischen 40 - 60 %. Besonders bevorzugt sind erfindungsgemäße Extrakte mit einem hohen Flavonoidgehalt. Als Extrakte zur pharmazeutischen und/oder kosmetischen Verwendung eignen sich nicht nur die nach dem erfindungsgemäßen Verfahren hergestellten Extrakte. Diese sind aber besonders bevorzugt.

Sofern die Extrakte für die erfindungsgemäße pharmazeutische oder kosmetische Verwendung nicht nach dem erfindungsgemäßen Verfahren hergestellt werden, können Sie durch Verfahren erhalten werden, die dem Fachmann an sich bekannt sind.

Die kosmetischen oder pharmazeutischen Zubereitungen, insbesondere die dermatologischen Zubereitungen der Erfindung, enthalten bevorzugt 0,001 % - 10 %, vorzugsweise 0,01% - 10%, bevorzugter 0,1% - 10%, am bevorzugtesten 1 - 5 % Gewichtsanteil der genannten Extrakte von *Reseda luteola* oder anderen Reseda-Arten, in Bezug auf das Gesamtgewicht der Zubereitung.

Die genannten Zubereitungen der Erfindung können vorteilhaft weitere Zusätze enthalten, im besonderen mindestens eine Substanz, die die Synthese von Bausteinen der extrazellulären Matrix der Haut stimulieren. Beispiele für solche Substanzen sind Vitamine, insbesondere Vitamine der A und C Gruppe und Derivate davon, Tocopherol, Xanthine, insbesondere Koffein oder Theophyllin, und Retinoide, insbesondere Vitamin A-Säure. Vorzugsweise zugesetzt werden können auch Pflanzenextrakte, wie Extrakte aus *Usnea barbata, Viola tricolor, Calendula officinalis, Rosmarinus officinalis,* Salvia-Arten, *Coriandrum sativum* und *Iris germanica.* Weitere Zusätze können bestehen aus Lichtschutzfiltem, zum Beispiel Titanoxiden, und pflanzlichen Lichtschutzfiltern. Der Extrakt aus *Reseda luteola* oder anderen Reseda-Arten, der zur erfindungsgemäßen Behandlung in Zubereitungen zur Linderung von Hautentzündungen, insbesondere Neurodermitis, verwendet wird, kann vorteilsweise kombiniert werden mit mindestens einem anderen aktiven Prinzip, das die Funktion der Hautbarriere fördert, wie Extrakten aus *Potentilla erecta* oder anderen Gerbstoffzubereitungen. Außerdem können die erfindungsgemäßen Reseda-Extrakte vorteilhaft zur Stabilisierung anderer oxidationsempfindlicher Substanzen verwendet werden. Besonders vorteilhaft ist zum Beispiel die Kombination von Hyperforin oder einem Hyperforin-angereicherten Hypericum-Extrakt mit erfindungsgemäßen Reseda-Extrakten.

Die pharmazeutische oder kosmetische Zusammensetzung der Erfindung kann auch eine pharmazeutisch bzw. kosmetisch verträgliche Trägersubstanz enthalten. Üblicherweise ist die Trägersubstanz für die topische Anwendung geeignet, sie beeinträchtigt nicht die Wirkung der Wirkstoffe und ist toxikologisch unbedenklich. Geeignete pharmazeutische Trägersubstanzen sind beschrieben in Remington's Pharmaceutical Sciences, 17th ed., Mack Publishing Company, Easton, Pa. (1990), einem Standardlehrbuch auf diesem Gebiet. Die Konzentration des Trägers in der Zusammensetzung ist nicht besonders eingeschränkt. Sie kann 5%-99,99% betragen, vorzugsweise 25 bis 99,9%, bevorzugter 50 bis 99%, am bevorzugtesten 50 bis 95%, bezogen auf die Gesamtzusammensetzung.

Die Zusammensetzung kann weitere optionale Substanzen enthalten. Das CTFA Cosmetic Ingredient Handbook, Second Edition (1992) beschreibt zahlreiche kosmetische und pharmazeutische Zusatzstoffe, die in der Industrie für Hautpflegeprodukte und Arzneimittel für die Haut eingesetzt werden.

Die erfindungsgemäßen Produkte können mit verschiedenen galenischen Zubereitungen hergestellt werden. Eine der gebräuchlichsten Zubereitungen ist eine topische Zubereitung, die für die Anwendung auf der Haut formuliert ist. Diese brauchbaren topischen Formulierungen schließen, ohne exklusiv zu sein, Salben, Emulsionen, Cremes, Pasten, Milch, Balsam, Gele, Lotionen, Tinkturen, Pflaster, Puder, Sprays, Schäume und Make up-Kompositionen ein. Verschiedene Verfahren zur Herstellung topischer Formulierungen sind dem Fachmann an sich bekannt. Sie sind im Detail beschrieben in Remington's Pharmaceutical Sciences (s.o.) oder in Pharmaceutical Dosage Forms and Drug Delivery Systems, 6th ed., Williams & Wilkins (1995).

Die Applikation der Zubereitungsformen erfolgt vorzugsweise zweimal täglich, indem die Zubereitungen dünn aufgetragen und leicht einmassiert werden. Wegen der guten Verträglichkeit ist die Anwendungsdauer nicht besonders eingeschränkt.

Die erfindungsgemäße Verwendung von Reseda-Extrakten bezieht sich dementsprechend auf die Anwendung eines kosmetisch, respektive pharmazeutisch effektiven Anteils von *Reseda luteola* oder anderen Reseda-Arten, wobei der bezeichnete Extrakt in eine Zubereitung mit einem kosmetisch, respektive pharmazeutisch verträglichen Carrier eingearbeitet wird.

Die so beschriebenen Methoden ermöglichen die verträgliche und wirksame Linderung von Hautreizungen, die Straffung und Glättung der schlaffen und faltigen Haut, die Vorbeugung oder Verminderung von Lichtschäden und die Behandlung von Sonnenbrand und Neurodermitis. Die Erfindung betrifft ein Verfahren zur Behandlung oder Vorbeugung von unerwünschten Zuständen der Haut, wobei einem Individuum, vorzugsweise einem Menschen, eine wirksame Menge eines Extrakts wenigstens einer Pflanze der Familie der Resedaceae verabreicht wird. Weiterhin betrifft die Erfindung ein Verfahren zur Hemmung der Proliferation aktivierter mononukleärer Zellen (Lymphozyten), ein Verfahren zur Induktion von Apoptose, vorzugsweise in aktivierten mononukleären Zellen (Lymphozyten), ein Verfahren zur Hemmung der Cyclooxygenase, ein Verfahren zur Stimulation von Fibroblasten und/oder ein Verfahren zur Hemmung der UVA-1-induzierten Toxizität, wobei jeweils einem Individuum eine wirksame Menge eines Extrakts wenigstens einer Pflanze der Familie der Resedaceae verabreicht wird. Die verschiedenen Ausführungsformen in dieser Anmeldung können miteinander kombiniert werden. Andere Ansprüche, Eigenschaften und Vorteile der Erfindung sind für den auf dem beschriebenen Gebiet Erfahrenen leicht aus den Beispielen ersichtlich.

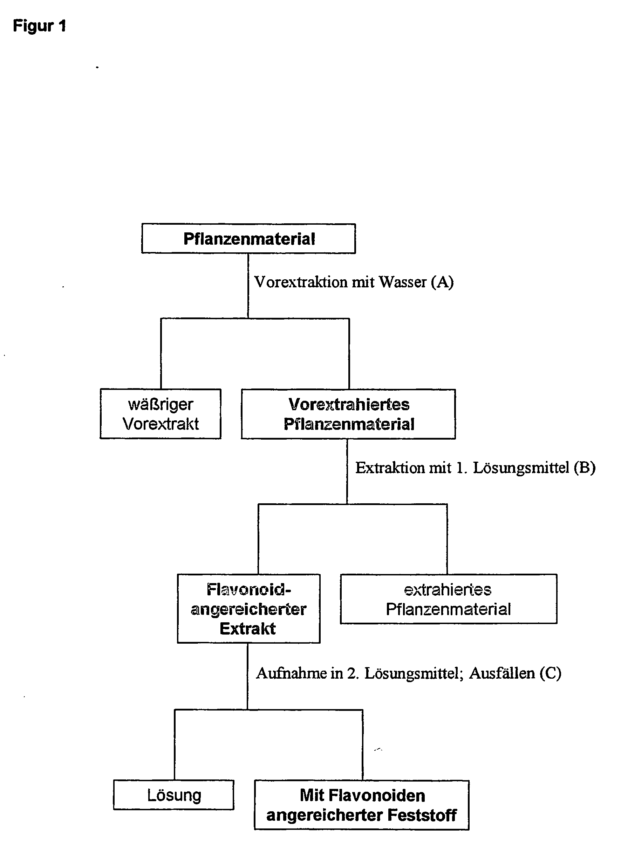

Figur 1 zeigt schematisch den Ablauf des erfindungsgemäßen Verfahrens in einer bestimmten Ausführungsform. In Schritt A) findet eine Vorextraktion von Pflanzenmaterial mit Wasser statt. Das vorextrahierte Pflanzenmaterial wird in Schritt B) einer Extraktion mit einem ersten Lösungsmittel unterworfen. Der erhaltene Extrakt wird konzentriert und in Schritt C) in einem 2. Lösungsmittel aufgenommen. Nach dem Ausfällen erhält man einen Feststoff, der mit Flavonoiden angereichert ist.

Figur 2 zeigt eine schematische Darstellung verschiedener Varianten des Verfahrens zur Herstellung eines Reseda-Extrakts.

Figur 3a zeigt die Hemmung der Proliferation aktivierter mononukleärer Zellen. Figur 3b zeigt, dass Luteolin die stärkste proliferationshemmende Wirkung aufweist (Beispiel 6).

Figur 4 zeigt die Hemmung der Cyclooxygenasen COX-1 und COX-2 im Vergleich zu Indomethacin (Beispiel 7).

Figur 5a zeigt die Induktion von Apoptose in aktivierten mononukleären Zellen durch Reseda-Extrakt. Aus Figur 5b ist ersichtlich, daß Luteolin eine starke apoptoseinduzierende Wirkung zeigte (Beispiel 12).

Figur 6a zeigt die Stimulation der Proliferation von Fibroblasten durch Reseda-Extrakt. In Figur 6b ist gezeigt, daß der Resedaextrakt zu einer Zunahme der Kollagensynthese führt (Beispiel 14).

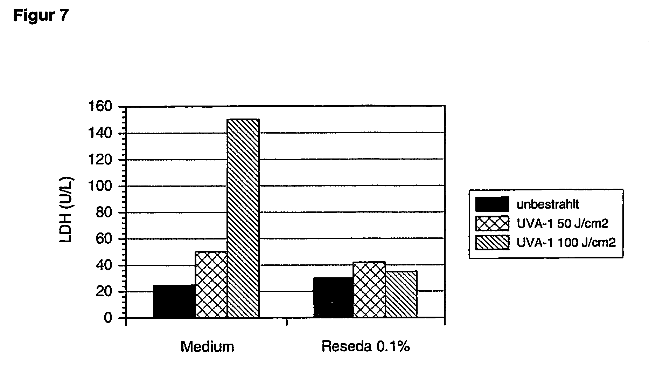

In Figur 7 ist die Hemmung der UV-induzierten Toxizität durch Reseda-Extrakt dargestellt (Beispiel 15).

Die nachfolgenden Beispiele erläutern die Erfindung näher und schränken diese in keiner Weise ein. Anhand von experimentellen und klinischen Beispielen werden die Anwendungsmöglichkeiten und das pharmazeutische bzw. kosmetologische Potential der Resedaextrakte verdeutlicht.

### Vergleichsbeispiel 1: Herstellung eines Wasserextraktes

Die oberirdischen Pflanzenteile von Reseda werden in getrocknetem und zerkleinertem Zustand im Verhältnis von ca. 1:5 mit Wasser bei Raumtemperatur extrahiert. Der Extrakt wird filtriert, konzentriert und oder getrocknet. Das erhaltene Pulver enthält ca. 0,5% - 1,5% Luteolin.

### Vergleichsbeispiel 2: Herstellung eines Wasserextraktes unter Zugabe von Alkalien

Die oberirdischen Pflanzenteile von Reseda werden in getrocknetem und zerkleinertem Zustand mit Wasser unter Zugabe von Alkalien bei einem pH-Wert von 7-9 bei einer Temperatur von 40°C erschöpfend extrahiert. Der Extrakt wird filtriert, konzentriert und oder getrocknet. Das erhaltene Pulver enthält ca. 2,5% - 5% Luteolin.

**Vergleichsbeispiel 3:** Herstellung eines Lösungsmittelextraktes aus Reseda

Die oberirdischen Pflanzenteile von Reseda werden in getrocknetem und zerkleinertem Zustand mit einem 50%igen Ethanol/Wasser-Gemisch bei einer Temperatur von 50°C erschöpfend extrahiert. Aus dem filtrierten Extrakt wird der Alkohol zurückgewonnen. Die erhaltene wäßrige Phase wird konzentriert und getrocknet. Das erhaltene Pulver enthält ca. 5% 10% Luteolin.

### Beispiel 1: Herstellung eines Luteolin-angereicherten Resedaextraktes

Die oberirdischen Pflanzenteile von Reseda werden in getrocknetem und zerkleinertem Zustand im Verhältnis von ca. 1:5 mit Wasser bei Raumtemperatur extrahiert. Der Extrakt wird filtriert, konzentriert und getrocknet Das erhaltene Pulver enthält ca. 0,5% - 1,5% Luteolin.
Die wasservorextrahierten Pflanzenteile werden mit einem 60%igen Alkohol/Wassergemisch bei einer Temperatur von 50°C erschöpfend extrahiert. Aus dem filtrierten Extrakt wird der Alkohol zurückgewonnen. Die erhaltene wäßrige Phase wird konzentriert und getrocknet. Das erhaltene Pulver enthält ca. 10% - 15% Luteolin.

### Beispiel 2: Darstellung eines höher Luteolin-angereicherten Resedaextraktes

Das in Vergleichsbeispiel 3 erhaltene Pulver wird mit Wasser im Verhältnis 1:5 bei Siedetemperatur aufgenommen. Nach dem Abkühlen der Lösung bildet sich ein Niederschlag, der durch Separieren, Dekantieren oder Filtrieren gewonnen werden kann. Die Niederschlag wird getrocknet. Das Pulver enthält mindestens 25% Luteolin.

### Beispiel 3: Darstellung eines höher Luteolin-angereicherten Resedaextraktes

Das in Beispiel 1 erhaltene Pulver wird mit einem 50%igen Alkohol/Wassergemisch im Verhältnis 1:7 bei 60°C aufgenommen. Die erhaltene Zusammensetzung wird zur Abtrennung des Alkohols destilliert und mit Wasser 1:1 verdünnt. Nach dem Abkühlen bildet sich ein Niederschlag, der durch Separieren, Dekantieren oder Filtrieren gewonnen werden kann. Die Niederschlag wird getrocknet. Das Pulver enthält mindestens 25% Luteolin.

### Beispiel 4: Darstellung eines höher Luteolin-angereicherten Resedaextraktes

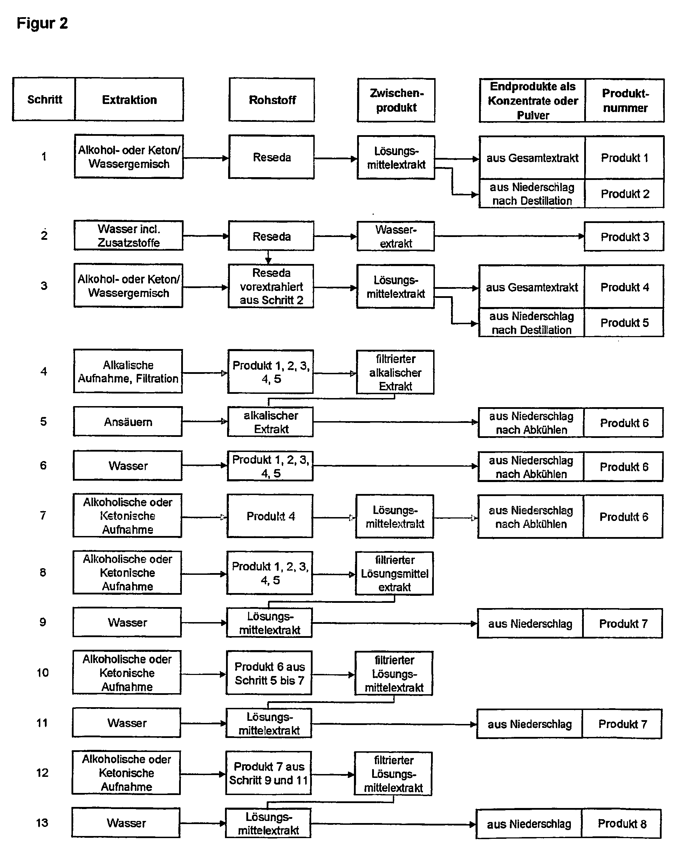

Die beschriebenen Produkte 1, 2, 4 oder 5 (siehe Figur 2) werden mit Wasser im Verhältnis 1:5 bei einer Temperatur von 70°C aufgenommen. Durch Zugabe von NaOH wird der pH-Wert auf 7 eingestellt. Der Extrakt wird heiß filtriert und durch Zugabe von HCl auf einen pH-Wert von 4 eingestellt. Nach dem Abkühlen der Lösung bildet sich ein Niederschlag, der durch Separieren, Dekantieren oder Filtrieren gewonnen werden kann. Die Niederschlag wird getrocknet. Das Pulver enthält mindestens 25% Luteolin.

### Beispiel 5: Darstellung eines höher Luteolin-angereicherten Resedaextraktes

Die beschriebenen Produkte 1, 2, 4 oder 5 (siehe Figur 2) werden im Verhältnis von 1:7 mit Alkohol oder Keton bei Siedetemperatur aufgenommen. Die Lösung wird in heißem Zustand filtriert.
Das Filtrat wird abgekühlt. Der abgekühlten alkoholischen Lösung wird zur Kristallisation der Flavonoide im Verhältnis von 1:3 Wasser zugesetzt. Die Mischung wird auf Temperaturen von 5 - 10°C gekühlt. Es bildet sich eine Niederschlag, der wie beschrieben gewonnen werden kann. Der getrocknete Niederschlag enthält mindestens 30% Luteolin. Eine weitere Reinigung erfolgt durch die erneute Umkristallisation des Niederschlages aus Alkohol mit Wasser. Das wie beschrieben gewonnene Pulver enthält mindestens 40% Luteolin.

### Beispiel 6: Hemmung der Proliferation stimulierter mononukleärer Zellen durch Reseda luteola-Extrakte:

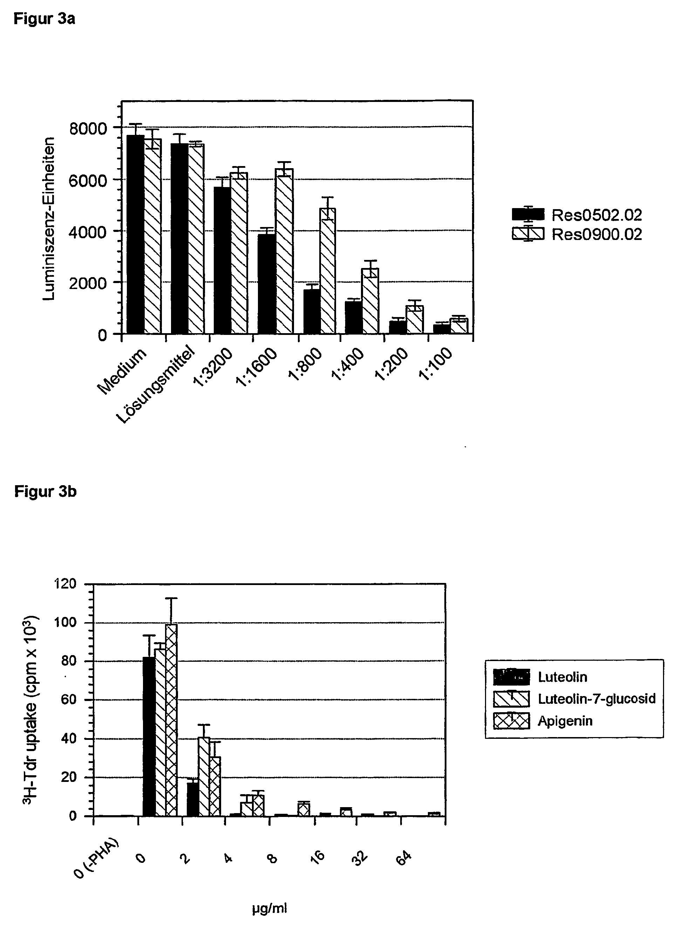

Der Test wurde mit den erfindungsgemäßen Extrakten nach Beispiel 1 (Extrakt Res0900.02) und Beispiel 3 (Extrakt Res0502.02) durchgeführt. Mononukleäre Zellen wurden aus dem peripheren Blut eines gesunden männlichen Spenders über Ficoll-Dichtegradienten gewonnen und in RPMI-Medium mit 5% fetalem Kälberserum in Mikrotiterplatten kultiviert. Die Zellen wurden in Triplikaten mit Medium (Negativkontrolle), Lösungsmittel (DMSO) oder mit in DMSO gelösten Reseda-Extrakten in den Konzentrationen 1:100, 1:200, 1:400, 1:800, 1:1600 und 1:3200 inkubiert. Nach Zugabe der Zusätze wurden die Zellen mit 1 µg/ml Phytohämagglutinin stimuliert und für 48 h im Brutschrank inkubiert. Dann wurde die Zellproliferation über den ATP-Gehalt der Zellen bestimmt (Via-Light-Test). Es zeigte sich eine signifikante, dosisabhängige Hemmung der Zellproliferation durch die *Reseda luteola*-Extrakte. Der nach Beispiel 3 hergestellte Extrakt war deutlich wirksamer als der nach Beispiel 1 hergestellte Extrakt. Das Lösungsmittel hatte keine Einfluß auf die Proliferation (Figur 3a).

Bei der Untersuchung der einzelnen in den Extrakten enthaltenen Flavonoide zeigte sich, daß das Luteolin die stärkste proliferationshemmende Wirkung aufweist (Figur 3b). Dazu wurden mononukleäre Zellen aus dem peripheren Blut einer gesunden weiblichen Spenderin über Ficoll-Dichtegradienten isoliert und in RPMI-Medium mit 5% fetalem Kälberserum in Mikrotiterplatten inkubiert. Die Zellen wurden in Triplikaten mit den Flavonoiden Luteolin, Luteolin-7-Glucosid und Apigenin inkubiert. Die Reinsubstanzen wurden in 70% Ethanol gelöst und in Medium weiterverdünnt. Die Endkonzentrationen betrugen 64, 32, 16, 8, 4, 2 und 0 µg/mL. Nach Zusatz der Flavonoide wurden die Zellen mit 1 µg/mL Phytohämagglutinin stimuliert und für 24 h im Brutschrank bei 37°C inkubiert. Nach Zugabe von 1 µCurie radioaktiven ³H-Thymidins wurden die Zellen für weitere 18 h inkubiert, und anschließend wurde die inkorporierte Radioaktivität im Szintillationscounter (Canberra-Packard) gemessen.

### Beispiel 7: Hemmung der Cyclooxygenase-1 (COX-1) und COX-2 durch den erfindungsgemäßen Extrakt nach Beispiel 3 (Res0502.02) im Vergleich zu dem nichtsteroidalen Antiphlogistikum Indomethacin

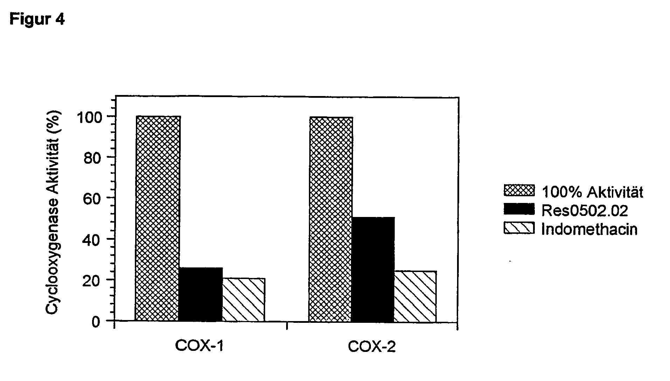

Als Nachweismethode dient ein zellfreier Enzym-Inhibitionskit der Firma Cayman Chemicals (Produktnummer #760111). Die Durchführung des Assays erfolgt bei 25°C, um optimale Enzymfunktion zu gewährleisten. Zu einem definierten Häm-haltigen Assaypuffer wird rekombinante COX-1 oder COX-2 gegeben. Anschließend wird Resedaextrakt Res0502.02 gemäß Beispiel 3 in einer Endkonzentration von 1:200, bzw. Indomethacin (Endkonz. 10 µM) und ein Farbsubstrat dazugegeben. Nach Zugabe des Enzymsubstrates Arachidonsäure läuft die Reaktion fünf Minuten, und die an die Bildung des Endoperoxides PGH2 gekoppelte Umsetzung des Farbsubstrates wird colorimetrisch gemessen. Die unbehandelte Enzymreaktion wird als 100%-Aktivität definiert und die erzielte Hemmung in Relation hierzu angegeben. Es zeigt sich, daß der Reseda-Extrakt eine dem Indomethacin vergleichbare Hemmung von COX-1, und etwas schwächer COX-2 bewirkt (Figur 4).

### Beispiel 8: Heilende Wirkung von Reseda luteola Creme bei der Behandlung von Neurodermitis.

a) Ein 31-jähriger Mann mit einer seit mehreren Wochen bestehenden Neurodermitis der Unterarme wurde mit der unter Beispiel 9 aufgeführten *Reseda luteola* Creme über 10 Tage behandelt. Hierunter kam es zu einer fast vollständigen Abheilung der Hautveränderungen. Außerdem verringerte sich die Faltentiefe, und die Straffheit der Haut nahm zu.
b) Ein 22-jähriger Mann mit einer Neurodermitis der Ellenbeuge wurde eine Woche mit der unter Beispiel 9 beschriebenen *Reseda lufeola* Creme behandelt. Bei der Kontrolle hatte sich die Rötung fast vollständig zurückgebildet.
c) Eine 24-jährige Frau mit einer seit bereits vielen Jahren bestehenden therapieresistenten Neurodermitis wurde mit der unter Beispiel 9 beschriebenen *Reseda luteola* Creme im Vergleich zu der stark entzündungshemmenden Protopic® Salbe 0, 1 % für zwei Wochen behandelt. Die *Reseda luteola* Creme wurde am rechten Handgelenk aufgetragen, die Protopic® Creme am linken Handgelenk. Bei der Kontrolle zeigte sich auf der mit Resedea luteola Creme behandelten Haut eine Besserung des Befundes, die der mit Protopic® behandelten Seite entsprach. Auf der mit Reseda lueola behandelten Seite zeigte sich zusätzlich eine deutliche Verminderung der Faltentiefe und eine Straffung der Haut.
d) Eine 68-jährige Frau litt an einem Ekzem im Gesicht mit Trockenheit, Schuppung und schmerzhaften Rhagaden. Nach 10 Tagen Behandlung mit Reseda luteola Creme gemäß Beispiel 9 war es zu einer Abheilung der Rhagaden und zu einem deutlichen Rückgang von Erythem und Schuppung gekommen.

### Beispiel 9: Herstellung einer Reseda luteola Creme zur Intensivpflege entzündeter Haut.

| | |
|---|---|
| Extrakt aus *Reseda luteola* gemäß Beispiel 3 | 2,0 |
| Glycerolmonostearat 60 | 4,0 |
| Cetylalkohol | 6,0 |
| Mittelkettige Triglyceride | 7,5 |
| Squalan | 25,5 |
| Macrogol-20-Glycerolmonostearat | 7,0 |
| Propylenglykol | 10,0 |
| Gereinigtes Wasser | ad. 100 |

Die Bestandteile neben dem Resedaextrakt bilden die Cremegrundlage. Die Bestandteile der Cremegrundlage können in anderen Mengenverhältnissen als den angegebenen enthalten sein. Die Cremegrundlage kann andere und/oder weitere Substanzen umfassen.

### Beispiel 10: Kosmetisches Pflegegel zur Behandlung von Falten und Hautreizungen im Gesicht.

| | |
|---|---|
| Wäßriger Extrakt aus *Reseda luteola* gemäß Beispiel 3 | 1,0 g |
| Extrakt aus *Potentilla erecta* | 0,5 g |
| Carbomer 50.000 | 0,5 g |
| 2-Propanol | 5,0 g |
| Propylenglykol | 10,0 g |
| Natriumhydroxid | 0,12 g |
| Gereinigtes Wasser | ad 100 |

Die Bestandteile neben den Pflanzenextrakten bilden die Gelgrundlage. Die Gelgrundlage kann die Bestandteile auch in anderen Mengenverhältnissen enthalten. Sie kann andere und/oder weitere Substanzen enthalten.

### Beispiel 11: Spezialsalbe zur Behandlung trockener Hautzustände.

| | |
|---|---|
| Extrakt aus *Reseda luteola* gemäß Beispiel 3 | 1,0 g |
| *Calendula officinalis* CO2- Extrakt | 3,0 g |
| Natriumdodecylsulfat | 5,0 g |
| Mittelkettige Triglyceride | 5,0 g |
| Glycerolmonostearat 60 | 20,0 g |
| Carnaubawachs | 20,0 g |
| Squalan | 25,0 g |
| 2-Octyldodecanol | 25,0 g |

Die Bestandteile neben den Pflanzenextrakten bilden die Salbengrundlage. Die Salbengrundlage kann die Bestandteile auch in anderen Mengenverhältnissen enthalten. Sie kann andere und/oder weitere Substanzen enthalten.

### Beispiel 12: Induktion von Apoptose in aktivierten mononukleären Zellen (Lymphozyten)

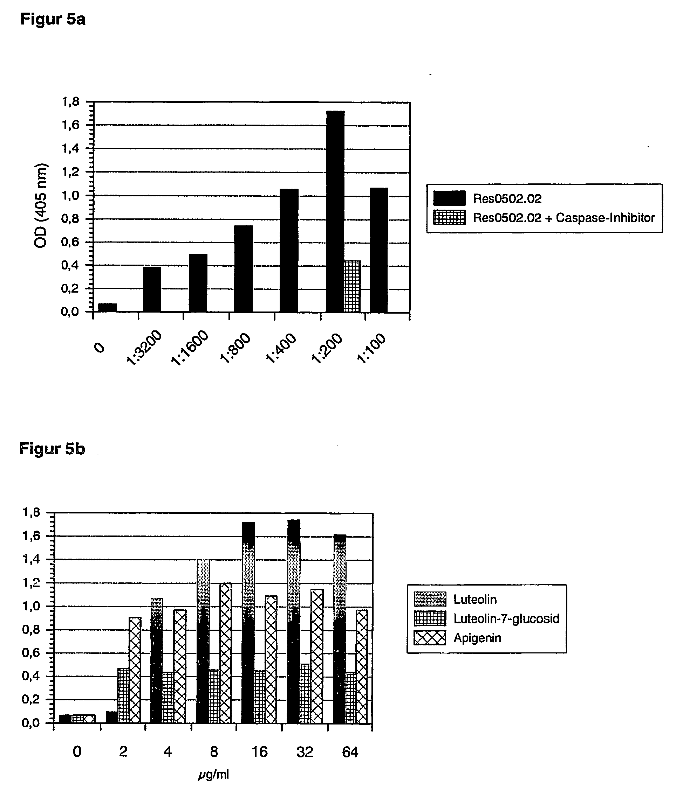

Mononukleäre Zellen (Lymphozyten) wurden mit Lösungsmittel, Medium und mit luteolinangereicherten Resedaextrakten gemäß Beispiel 3 in den Konzentrationen 1:1000 (100 µg/mL), 1:200 (50 µg/mL), 1:400 (25 µg/mL), 1:800 (12,5 µg/mL), 1:1600 (6,25 µg/mL und 1:3200 (3,125 µg/mL) inkubiert. "0" in Figur 5a bedeutet, daß kein Resedaextrakt anwesend war. Nach Zugabe der Zusätze wurden die Zellen mit 1 µg/mL Phytohämagglutinnin stimuliert und für 48 h im Brutschrank inkubiert. Dann wurde die Apoptoseinduktion durch Messung der in den Zellen entstandenen Oligonukleosomen (niedermolekulare DNA-Fragmente) bestimmt (Cell Death detection ELISA). Es zeigte sich eine signifikante, dosisabhängige Steigerung der Apoptoserate durch die Extrakte. Die Apoptoseinduktion war durch einen Caspaseinhibitor blockierbar, ist also Caspase-induziert (Figur 5a). Bei der Untersuchung der einzelnen in den Extrakten enthaltenen Flavonoide zeigte sich, daß das Luteolin die stärkste Apoptose-induzierende Wirkung aufweist (Figur 5b).

### Beispiel 13: Antioxidative Wirkung von Resedaextrakt

Die relative antioxidative Wirkung von Resedaextrakt wurde nach der TEAC III-Methode im Vergleich zu Trolox und Ascorbinsäure bestimmt:
- Trolox: 1,0
- Ascorbinsäure 1,1
- Resedaextrakt 1,5

Die TEAC III-Methode und andere Verfahren werden in Böhm V. (2000) Ernährungsumschau 47, S. 372-375 beschrieben.

### Beispiel 14: Stimulation von Fibroblasten

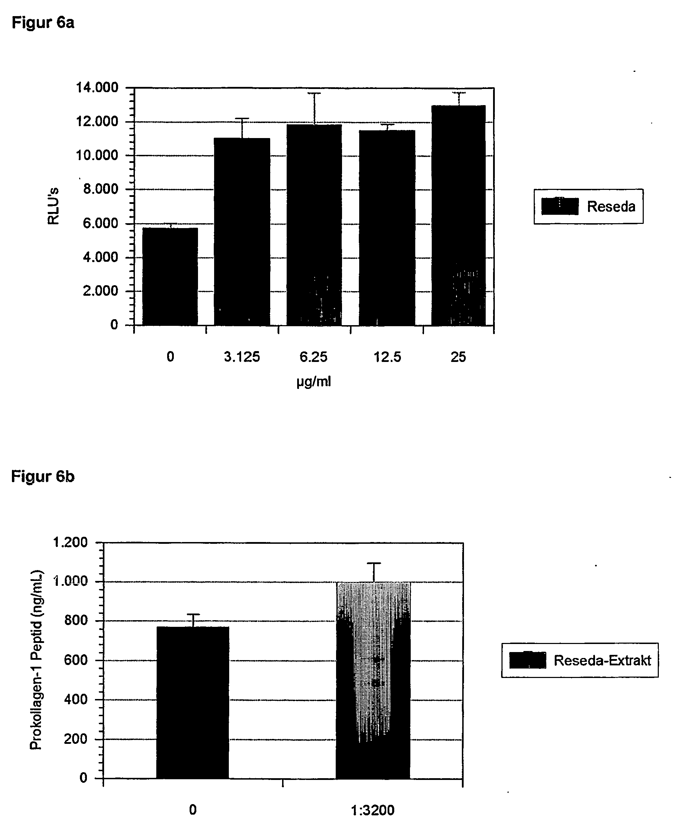

In einem weiteren Test wurde die Wirkung eines luteolinangereicherten Resedaextrakts auf die Proliferation von primären humanen Fibroblasten untersucht. Es zeigte sich überraschend, daß der Extrakt in sehr niedrigen Konzentrationen die Proliferation der Fibroblasten stimuliert (ViaLight Assay) (Figur 6a) und zu einer Zunahme der Kollagensynthese führt (Prokollagen 1 C Peptid EIA) (Figur 6b). Es wurde der Resedaextrakt Nr. 3 = Res0502.02 eingesetzt. Die Konzentrationen 25 µg/mL, 12,5 µg/mL, 6,25 µg/mL und 3,125 µg/mL entsprechen den Verdünnungen 1:400, 1:800, 1:1600 bzw. 1:3200. Während in Figur 6a die Konzentrationen aufgetragen sind, ist in Figur 6b die Verdünnung des Resedaextrakts angegeben. "0" in Figur 6b bedeutet, daß kein Resedaextrakt anwesend war.

### Beispiel 15: Hemmung der UV-induzierten Toxizität durch Resedaextrakt Modell: LDH-Freisetzung aus primären humanen Fibroblasten

Subkonfluente primäre humane Fibroblasten wurden in PBS-Puffer mit 0/50/100 J/cm² UVA-1 bestrahlt. Anschließend wurden die Zellen in Zellkulturmedium mit und ohne Zusatz von Resedaextrakt (0,1%) für 24 h inkubiert. Im Überstand wurde dann die Freisetzung von Lactat-Dehydrogenase (LDH) gemessen (Mira-Cobas Enzymtest). Der Zusatz von Resedaextrakt in niedriger Konzentration hemmt die UVA-1-induzierte Schädigung der Zellmembran fast vollständig. Es wurde Resedaextrakt gemäß Beispiel 3 eingesetzt (Res0502.02). Die Konzentration 0, 1 % entspricht einer Verdünnung von 1:1000.

## Patentansprüche

1. Verfahren zur Herstellung einer flavonoidhaltigen Zusammensetzung, das umfasst, dass man
a) Material wenigstens einer Pflanze der Familie der Resedaceae einer Vorextraktion mit einer wässrigen Lösung unterzieht,
b) den in Schritt a) erhaltenen Rückstand an Pflanzenmaterial mit einem ersten Lösungsmittel extrahiert, das eine Lösung mit einem Anteil von 15 bis 100 % an polarem organischen Lösungsmittel in Wasser ist, und
c) wenigstens einen Teil des ersten Lösungsmittels aus dem in Schritt b) erhaltenen Extrakt entfernt, um eine Zusammensetzung mit einem Luteolingehalt von wenigstens 10 % zu erhalten.

2. Verfahren nach Anspruch 1, **dadurch gekennzeichnet, daß** Material der Pflanzensorte *Reseda luteola L.* eingesetzt wird.

3. Verfahren nach Anspruch 1 oder 2, **dadurch gekennzeichnet, daß** das erste Lösungsmittel ein Wasser/Alkohol- oder ein Wasser/Keton-Gemisch ist.

4. Verfahren nach einem der Ansprüche 1 bis 3, **dadurch gekennzeichnet, daß** in Schritt c) eine Destillation des in Schritt b) erhaltenen Extrakts durchgeführt wird.

5. Verfahren nach einem der Ansprüche 1 bis 4, **dadurch gekennzeichnet, daß** es weiterhin umfaßt, daß man
d) das in Schritt c) erhaltene Produkt in einem zweiten Lösungsmittel aufnimmt und gegebenenfalls unlösliche Bestandteile abtrennt; und
e) die in Schritt d) erhaltene Lösung ansäuert, abkühlt und/oder mit Wasser versetzt, so daß ein Feststoff erhalten wird.

6. Verfahren nach Anspruch 5, **dadurch gekennzeichnet, daß** das in Schritt c) erhaltene Produkt in einer wäßrigen Lösung mit einem pH-Wert von 6 bis 10 aufgenommen wird, die unlöslichen Bestandteile abgetrennt werden, die so erhaltene Lösung auf einen pH-Wert von 2,5 bis 6 eingestellt wird und der sich bildende Feststoff gewonnen wird.

7. Verfahren nach Anspruch 5, **dadurch gekennzeichnet, daß** das in Schritt c) erhaltene Produkt in Wasser mit einer Temperatur von 30 bis 100°C aufgenommen wird, die entstehende Lösung abgekühlt wird und der sich bildende Feststoff gewonnen wird.

8. Verfahren nach Anspruch 5, **dadurch gekennzeichnet, daß** das in Schritt c) erhaltene Produkt in einem Wasser/Alkohol- oder einem Wasser/Keton-Gemisch mit einer Temperatur von 20 bis 80 °C aufgenommen wird, Wasser zugegeben wird und der sich bildende Feststoff gewonnen wird.

9. Verfahren nach Anspruch 5, **dadurch gekennzeichnet, daß** das in Schritt c) erhaltene Produkt mit einem Wasser/Alkohol- oder Wasser/Keton-Gemisch aufgenommen wird, unlösliche Bestandteile entfernt werden, anschließend Wasser zugegeben wird und der sich bildende Feststoff gewonnen wird.

10. Verfahren nach einem der Ansprüche 5 bis 9, **dadurch gekennzeichnet, daß** der in Schritt e) erhaltene Feststoff mit einem Wasser/Alkohol- oder einem Wasser/Keton-Gemisch aufgenommen wird, unlösliche Bestandteile entfernt werden, Wasser zugegeben wird und der sich bildende Feststoff gewonnen wird.

11. Verfahren nach Anspruch 10, **dadurch gekennzeichnet, daß** der erhaltene Feststoff nochmals mit einem Wasser/Alkohol- oder ein Wasser/Keton-Gemisch aufgenommen wird, unlösliche Bestandteile entfernt werden, Wasser zugegeben wird und der sich bildende Feststoff gewonnen wird.

12. Verfahren zur Herstellung einer Flavonoid-haltigen Zusammensetzung mit einem Luteolingehalt von wenigstens 10 %, das umfasst, dass man
a) Material wenigstens einer Pflanze der Familie der Resedaceae mit einem ersten Lösungsmittel extrahiert, das eine Lösung mit einem Anteil von 15 bis 100 % an polarem organischen Lösungsmittel in Wasser ist,
b) wenigstens einen Teil des ersten Lösungsmittels aus dem in Schritt a) erhaltenen Extrakt entfernt;
c) das in Schritt b) erhaltene Produkt in einem zweiten Lösungsmittel aufnimmt und gegebenenfalls unlösliche Bestandteile abtrennt; und
d) die in Schritt c) erhaltene Lösung ansäuert, abkühlt und/oder mit Wasser versetzt, so daß ein Feststoff erhalten wird.

13. Verfahren nach Anspruch 12, **dadurch gekennzeichnet, daß** der in Schritt d) erhaltene Feststoff mit einem Wasser/Alkohol- oder einem Wasser/Keton-Gemisch aufgenommen wird, unlösliche Bestandteile entfernt werden, Wasser zugegeben wird und der sich bildende Feststoff gewonnen wird.

14. Verfahren nach Anspruch 13, **dadurch gekennzeichnet, daß** der erhaltene Feststoff mit einem Wasser/Alkohol- oder einem Wasser/Keton-Gemisch aufgenommen wird, unlösliche Bestandteile entfernt werden, Wasser zugegeben wird und der sich bildende Feststoff gewonnen wird.

15. Flavonoid-haltige Zusammensetzung erhältlich durch ein Verfahren nach einem der Ansprüche 1 bis 14.

16. Flavonoid-haltiger Pflanzenextrakt enthaltend wenigstens 10% Luteolin.

17. Verwendung einer Zusammensetzung nach Anspruch 15 oder eines Extrakts wenigstens einer Pflanze der Familie der Resedaceae zur Herstellung eines Medikaments zur Behandlung oder Vorbeugung von Neurodermitis, Lichen ruber, Prurigo, Psoriasis, Pemphigus, Pemphigoid, Dermatitis herpetiformis, Sklerodermie, Lichen sclerosus, Dermatitis solaris, Morbus Favre Racouchaud, aktinische Keratosen, Elastosis cutis, Akne vulgaris, Follikulitis simplex, Rosacea oder Sonnenbrand, oder zur Straffung und/oder Glättung der Haut, oder zur Vorbeugung von Faltenbildung und/oder Lichtalterung der Haut.

18. Verwendung nach Anspruch 17, **dadurch gekennzeichnet, daß** eine topische Formulierung verabreicht wird, die ausgewählt ist aus der Gruppe bestehend aus Salben, Emulsionen, Cremes, Pasten, Milch, Balsam, Gele, Lotionen, Tinkturen, Pflaster, Puder, Sprays, Schäume und Make up-Kompositionen.

19. Hautpflegeprodukt enthaltend eine Zusammensetzung nach Anspruch 15 oder einen Extrakt wenigstens einer Pflanze der Familie der Resedaceae.

20. Hautpflegeprodukt nach Anspruch 19, **dadurch gekennzeichnet, dass** es weiterhin einen weiteren Pflanzenextrakt enthält, der nicht von einer Pflanze der Familie der Resedaceae stammt.

21. Hautpflegeprodukt nach Anspruch 19 oder 20, **dadurch gekennzeichnet, daß** es ausgewählt ist aus der Gruppe bestehend aus Salben, Emulsionen, Cremes, Pasten, Milch, Balsam, Gele, Lotionen, Tinkturen, Pflaster, Puder, Sprays, Schäume und Make up-Kompositionen.

## Claims

1. Method for the production of a flavonoid-containing composition, which comprises
a) pre-extracting material of at least one plant of the Resedaceae family, with an aqueous solution,
b) extracting the residue of plant material obtained in step a) with a first solvent, which is a solution comprising 15 to 100 % of polar organic solvent in water, and
c) removing at least a part of the first solvent from the extract obtained in step b) in order to obtain a composition comprising at least 10 % of luteolin.

2. Method according to Claim 1, **characterized in that** material of the plant variety *Reseda luteola L*. is used.

3. Method according to Claim 1 or 2, **characterized in that** the first solvent is a water/alcohol or a water/ketone mixture.

4. Method according to any of Claims 1 to 3, **characterized in that** a distillation of the extract obtained in step b) is carried out in step c).

5. Method according to any of Claims 1 to 4, **characterized in that** it furthermore comprises
d) taking up the product obtained in step c) in a second solvent and optionally separating off insoluble components; and
e) acidifying, cooling and/or adding water to the solution obtained in step d) so that a solid is obtained.

6. Method according to Claim 5, **characterized in that** the product obtained in step c) is taken up in an aqueous solution having a pH of 6 to 10, the insoluble components are separated off, the solution thus obtained is adjusted to a pH of 2.5 to 6 and the resulting solid is recovered.

7. Method according to Claim 5, **characterized in that** the product obtained in step c) is taken up in water having a temperature of 30 to 100°C, the resulting solution is cooled and the resulting solid is recovered.

8. Method according to Claim 5, **characterized in that** the product obtained in step c) is taken up in a water/alcohol or a water/ketone mixture having a temperature of 20 to 80°C, water is added and the resulting solid is recovered.

9. Method according to Claim 5, **characterized in that** the product obtained in step c) is taken up in a water/alcohol or water/ketone mixture, insoluble components are removed, water is then added and the resulting solid is recovered.

10. Method according to any of Claims 5 to 9, **characterized in that** the solid obtained in step e) is taken up with a water/alcohol or a water/ketone mixture, insoluble components are removed, water is added and the resulting solid is recovered.

11. Method according to Claim 10, **characterized in that** the solid obtained is again taken up with a water/alcohol or a water/ketone mixture, insoluble components are removed, water is added and the resulting solid is recovered.

12. Method for the production of a flavonoid-containing composition, which comprises
a) extracting material of at least one family of the Resedaceae family with a first solvent, which is a solution comprising 15 to 100 % of polar organic solvent in water,
b) removing at least a part of the first solvent from the extract obtained in step a);
c) taking up the product obtained in step b) in a second solvent and optionally separating off insoluble components; and
d) acidifying, cooling and/or adding water to the solution obtained in step c) so that a solid is obtained.

13. Method according to Claim 12, **characterized in that** the solid obtained in step d) is taken up with a water/alcohol or a water/ketone mixture, insoluble components are removed, water is added and the resulting solid is recovered.

14. Method according to Claim 13, **characterized in that** the solid obtained is taken up with a water/alcohol or a water/ketone mixture, insoluble components are removed, water is added and the resulting solid is recovered.

15. Flavonoid-containing composition obtainable by a method according to any of Claims 1 to 14.

16. Flavonoid-containing plant extract comprising at least 10% of luteolin.

17. Use of a composition according to Claim 15 or of an extract of at least one plant of the Resedaceae family for the production of a medicament for treating or preventing neurodermatitis, Lichen ruber, prurigo, psoriasis, pemphigus, pemphigoid, Dermatitis herpetiformis, sclerodermatitis, Lichen sclerosis, Dermatitis solaris, Favre Racouchot syndrome, actinic keratoses, Elastosis cutis, common acne, Folliculitis simple, rosacea or sunburn, for tautening and/or smoothing the skin, or for preventing wrinkles and/or light-induced ageing of the skin.

18. Use according to Claim 17, **characterized in that** a topical formulation is administered, which formulation is selected from the group consisting of ointments, emulsions, creams, pastes, milk, balsam, gels, lotions, tinctures, plasters, powders, sprays, foams and make-up compositions.

19. Skin-care product comprising a composition according to Claim 15 or an extract of at least one plant of the Resedaceae family.

20. Skin-care product according to Claim 19, **characterized in that** it furthermore contains a further plant extract which does not originate from a plant of the Resedaceae family.

21. Skin-care product according to Claim 19 or 20, **characterized in that** it is selected from the group consisting of ointments, emulsions, creams, pastes, milk, balsam, gels, lotions, tinctures, plasters, powders, sprays, foams and make-up compositions.

## Revendications

1. Procédé pour préparer une composition contenant un flavonoïde, qui comprend les étapes consistant à :
a) soumettre le matériau d'au moins une plante de la famille des résédacées à une extraction préliminaire avec une solution aqueuse,
b) extraire le résidu de matériau végétal obtenu à l'étape a), avec un premier solvant représenté par une solution ayant une fraction de 15 à 100 % de solvant organique polaire dans de l'eau, et
c) éliminer au moins une partie du premier solvant de l'extrait obtenu à l'étape b), pour obtenir une composition présentant une teneur en lutéoline d'au moins 10 %.

2. Procédé selon la revendication 1, **caractérisé en ce que** l'on utilise un matériau de l'espèce végétale Reseda luteola L.

3. Procédé selon la revendication 1 ou 2, **caractérisé en ce que** le premier solvant est un mélange de type eau/alcool ou un mélange de type eau/cétone.

4. Procédé selon l'une quelconque des revendications 1 à 3, **caractérisé en ce que** l'on effectue à l'étape c) une distillation de l'extrait obtenu à l'étape b).

5. Procédé selon l'une quelconque des revendications 1 à 4, **caractérisé en ce qu'**il comprend également les étapes consistant à :
d) reprendre le produit obtenu à l'étape c) dans un second solvant et séparer les constituants éventuellement insolubles ; et
e) acidifier la solution obtenue à l'étape d), refroidir celle dernière et/ou la mélanger à de l'eau en vue d'obtenir un solide.

6. Procédé selon la revendication 5, **caractérisé en ce que** l'on reprend le produit obtenu à l'étape c) dans une solution aqueuse ayant une valeur de pH de 6 à 10, on sépare les constituants insolubles, on ajuste la solution ainsi obtenue à une valeur de pH de 2,5 à 6 et on récupère le solide en cours de formation.

7. Procédé selon la revendication 5, **caractérisé en ce que** l'on reprend le produit obtenu à l'étape c) dans de l'eau à une température de 30 à 100 °C, on refroidit la solution résultante et on récupère le solide en cours de formation.

8. Procédé selon la revendication 5, **caractérisé en ce que** l'on reprend le produit obtenu à l'étape c) dans un mélange eau/alcool ou dans un mélange eau/cétone à une température de 20 à 80 °C, on ajoute de l'eau et on récupère le solide en cours de formation.

9. Procédé selon la revendication 5, **caractérisé en ce que** l'on reprend le produit obtenu à l'étape c) dans un mélange eau/alcool ou dans un mélange eau/cétone, on élimine les constituants insolubles, puis on ajoute de l'eau et on récupère le solide en cours.de formation.

10. Procédé selon l'une quelconque des revendications 5 à 9, **caractérisé en ce que** l'on reprend le solide obtenu à l'étape e) dans un mélange eau/alcool ou dans un mélange eau/cétone, on élimine les constituants insolubles, on ajoute de l'eau et on récupère le solide en cours de formation.

11. Procédé selon la revendication 10, **caractérisé en ce que** l'on reprend une nouvelle fois le solide obtenu dans un mélange eau/alcool ou dans un mélange eau/cétone, on élimine les constituants insolubles, on ajoute de l'eau et on récupère le solide en cours de formation.

12. Procédé pour préparer une composition contenant un flavonoïde ayant une teneur en lutéoline d'au moins 10 %, qui comprend les étapes consistant à :
a) extraire un matériau d'au moins une plante de la famille des résédacées avec un premier solvant, lequel est représenté par une solution ayant une fraction de 15 à 100 % de solvant organique polaire dans de l'eau,
b) éliminer au moins une partie du premier solvant de l'extrait obtenu à l'étape a) ;
c) reprendre le produit obtenu à l'étape b) dans un second solvant et séparer les constituants éventuellement insolubles ; et
d) acidifier la solution obtenue à l'étape c), refroidir celle-ci et/ou la mélanger à de l'eau, de manière à obtenir un solide.

13. Procédé selon la revendication 12, **caractérisé en ce que** l'on reprend le solide obtenu à l'étape d) dans un mélange eau/alcool ou dans un mélange eau/cétone, on élimine les constituants insolubles, on ajoute de l'eau et on récupère le solide en cours de formation.

14. Procédé selon la revendication 13, **caractérisé en ce que** l'on reprend le solide obtenu dans un mélange eau/alcool ou dans un mélange eau/cétone, on sépare les constituants insolubles, on ajoute de l'eau et on récupère le solide en cours de formation.

15. Composition contenant un flavonoïde, que l'on peut obtenir par un procédé selon l'une quelconque des revendications 1 à 14.

16. Extrait végétal contenant un flavonoïde, contenant au moins 10 % de lutéoline.

17. Utilisation d'une composition selon la revendication 15, ou d'un extrait d'au moins une plante de la famille des résédacées, pour préparer un médicament en vue du traitement ou de la prévention de la neurodermatïte, du lichen ruber, du prurigo, du psoriasis, du pemphigus, de la pemphigoïde, de la dermatite herpétiforme, de la sclérodermie, du lichen sclerosus, de la dermatite solaire, de la maladie de Favre et Racouchaud, des kératoses actiniques, de l'élastose cutanée, de l'acné vulgaire, de la folliculite simplex, de l'acné rosacée ou du coup de soleil, ou pour raffermir et/ou lisser la peau, ou pour prévenir la formation de rides et/ou le vieillissement de la peau dû à la lumière.

18. Utilisation selon la revendication 17, **caractérisée en ce que** l'on administre une formulation topique, qui est choisie dans le groupe constitué des pommades, des émulsions, des crèmes, des pâtes, des laits, des baumes, des gels, des lotions, des teintures, des emplâtres, des poudres, des pulvérisations, des mousses et des compositions cosmétiques.

19. Produit de soin pour la peau, contenant une composition selon la revendication 15 ou un extrait d'au moins une plante de la famille des résédacées.

20. Produit de soin pour la peau selon la revendication 19, **caractérisé en ce qu'**il contient également un autre extrait végétal, qui ne provient pas d'une plante de la famille des résédacées.

21. Produit de soin pour la peau selon la revendication 19 ou 20, **caractérisé en ce que** l'on choisit ce dernier dans le groupe constitué des pommades, des émulsions, des crèmes, des pâtes, des laits, des baumes, des gels, des lotions, des teintures, des emplâtres, des poudres, des pulvérisations, des mousses et des compositions cosmétiques.
